(19) Europäisches Patentamt
European Patent Office
Office européen des brevets

(11) Publication number: 0 003 427 B1

(12) EUROPEAN PATENT SPECIFICATION

(45) Date of publication of patent specification: 14.10.81

(51) Int. Cl.³: C 07 C 47/55, C 07 C 45/00

(21) Application number: 79300120.7

(22) Date of filing: 24.01.79

(54) Preparation of m-(p-bromophenoxy)benzaldehyde.

(30) Priority: 25.01.78 JP 7760/78

(43) Date of publication of application: 08.08.79 Bulletin 79/16

(45) Publication of the grant of the European patent: 14.10.81 Bulletin 81/41

(84) Designated Contracting States: BE CH DE FR GB IT NL SE

(56) References cited: FR - A - 2 351 096

(73) Proprietor: SUMITOMO CHEMICAL COMPANY, LIMITED
15 Kitahama 5-chome Higashi-ku
Osaka-shi Osaka-fu (JP)

(72) Inventor: Ohsumi, Tadashi
196, Kanshadencho Higashiiru Takakura
Manjujidori Shimogyo-ku Kyoto (JP)
Inventor: Itaya, Nobushige
5-3-101, Ryodocho
Nishinomiya-shi (JP)

(74) Representative: Harrison, David Christopher
MEWBURN ELLIS & CO 70 & 72 Chancery Lane
London WC2A 1AD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 003 427 B1

Courier Press, Leamington Spa, England.

Preparation of m-(p-bromophenoxy)benzaldehyde

The present invention relates to a process for the production of m-(p-bromophenoxy)-benzaldehyde of the formula,

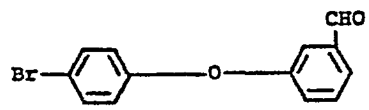

and more particularly it relates to the selective bromination of m-phenoxybenzaldehyde.

In general, the halogenation of aromatic hydrocarbons with a halogen is a well-known reaction, but this reaction requires a catalyst in many cases and produces isomers as by-products, the formation of which often makes the operation troublesome because of the need to separate the desired product therefrom. In particular, an industrial, direct halogenation process, carried out without protecting the highly reactive functional group, i.e. the aldehyde group, does not appear to have ever been achieved. In many cases, the halogenation is carried out after protecting the aldehyde group by forming a complex thereof with, for example, aluminium chloride.

As can be seen from, for example, the introduction to the paper of D. E. Pearsons: J. Org. Chem. *23*, 1412 (1958), it is common that the halogenation of aromatic aldehydes produces an acid halide when a catalyst is not used, while it produces a nuclear halogenated derivative only when a catalyst such as silver sulfate is used. The advance disclosed in that paper was that nuclear halogenation could take place under special conditions (1) an excess of aluminium chloride is used and this is firstly complexed with the aldehyde prior to halogenation, 2) no solvent is used and 3) the complex is sufficiently molten to be stirred during halogenation) but even then halogenation was only shown to have occurred on a keto- or aldehyde-substituted nucleus, e.g. acetophenone or benzaldehyde.

The present invention is concerned with the nuclear halogenation of the nucleus not substituted with aldehyde and is concerned specifically with bromination.

Further, in order to demonstrate that the bromination process of the present invention is one which makes maximum use of the structural characteristics of the compound itself, the chlorination of the compound was carried out for comparison under various conditions in a manner similar to processes embodying the present invention: in each such chlorination process, chlorine gas was passed through a solution of m-phenoxybenzaldehyde in a solvent. The results are shown in Table 1.

As can be seen from the Table, chlorination produces an isomer, m-(o-chlorophenoxy)-benzaldehyde, as by-product together with large amounts of impurities. The position is improved to some extent by lowering the reaction temperature, but the problems such as low yield of the desired compound and formation of m-(o-chlorophenoxy)benzaldehyde as by-product still remain unsolved. Particularly, the by-product, m-(o-chlorophenoxy)benzaldehyde formed is difficult to remove from the desired compound, m-(p-chlorophenoxy)benzaldehyde. For example purification by distillation is very difficult because of the similar boiling point of the both compounds, and chemical purification with sodium hydrogen sulfite cannot be an effective means because of almost identical behaviour of both compounds to that chemical. In order to improve the position concerning selectivity and yield, the catalytic effect of ferric chloride, zinc chloride and titanium chloride was investigated. Good results were not however obtained: The yield of pure substance was slightly improved but the amount of m-(o-chlorophenoxy)benzaldehyde increased, as is apparent from Table 1. Further, the effect of using different solvents was also investigated to some degree, from which it was found that chlorobenzene and tetrachloroethylene were inferior to methylene chloride in position selectivity and pure yield.

After our having previously found that the conventional pyrethroid type insecticides having a halogen atom at the p-position show a great reduction in toxicity to fish, we conducted extensive studies directed to obtaining m-phenoxybenzaldehyde having a halogen atom at the p-position, which is a useful intermediate for the production of these pyrethroid type insecticides (Kasamatsu et al.: Japanese Patent Application Nos. 71489/77 and 69119/77 (Unexamined Patent Publication Nos. 5936/79 and 5947/79)). As a result, it was found unexpectedly that the bromination of m-phenoxybenzaldehyde could, by carring out the process of the invention, be effected with an extremely high selectivity and a high yield and under conditions which enable the process to be carried out much more easily and economically than the abovementioned processes.

Thus there is no need to form complexes with aluminium chloride and indeed no catalyst at all need be used. In a preferred process embodying the invention a solvent is employed, and such a process can be carried out without heating.

m-Phenoxybenzaldehyde used as a starting material in processes embodying the present invention can easily be produced, for example by the processes disclosed in Japanese Patent Application Kokai (Laid-Open) Nos. 143638/1976, 40732/1978, 82736/1978 and 112824/1978. This compound is already commercially available as an intermediate for many pyrethroid type insecticides of

economical importance and extreme effectiveness. It is therefore apparent that the process of the present invention makes it possible to produce the objective compound at low cost and with extreme ease of operation, high yield and high selectivity as clearly shown in the examples, and therefore is far superior to other processes in economy and operation.

In the present invention, the bromination of m-phenoxybenzaldehyde may be carried out in the presence or absence of a solvent. However, it is preferably carried out in a halogenation-resistant solvent at a temperature lower than the boiling point of the solvent, preferably lower than room temperature, more preferably at —30°C to 20°C. The solvent includes methylene chloride, tetrachloroethylene, chlorobenzene and the like. Catalysts are not required at all in general, but the reaction is never hindered even though aluminium chloride, ferric chloride, zinc chloride as a catalyst in an amount of 0 to 5/100 mole per mole of m-phenoxybenzaldehyde is present in the system.

The completion of the reaction can easily be determined by the gas-chromatographical examination of the disappearance of the material. The objective m-(p-bromophenoxy)benzaldehyde can be obtained in a high purity and with high yield by merely removing the solvent by evaporation.

The results of the experimental bromination of m-phenoxybenzaldehyde according to the present invention are shown in Table 2. As is apparent from the table, the bromination proceeds smoothly without special heating even in the absence of a catalyst, and besides it shows an extremely high selectivity without being accompanied by m-(o-bromophenoxy)benzaldehyde as by-product.

Both an increase in the o-isomer and a remarkable effect on the pure yield are not observed unlike the chlorination, even though a metallic salt such as ferric chloride, aluminium chloride, zinc chloride or the like is present as the catalyst in the system. Further, in the present invention, tetrachloroethylene and chlorobenzene may be used as a solvent with the same good result as with methylene chloride.

The bromination of the present invention may be carried out using not only molecular bromine but also bromine chloride as a brominating agent.

The m-phenoxybenzaldehyde and the brominating agent are preferably used in a molar ratio of from 1:1 to 1:1.2. The brominating agent may be added to the reaction mixture over a period of from $\frac{1}{2}$ to 1 hour. After completion of the addition, the reaction may be continued for from 0.5 to 3 hours. The results are shown in Table 2.

Bromine chloride has the defect that the pure yield is low as compared with molecular bromine, but it is advantageous in cost and retention of the high selectivity. The crude product obtained by these methods can be purified into m-(p-bromophenoxy) benzaldehyde of higher purity by distillation or the like.

Next, the present invention will be illustrated in more detail with reference to the following reference example and examples. Examples and reference examples other than those mentioned below were carried out and their results are shown in Tables 1 and 2.

### Reference Example 1

9.90 Grams (0.50 mole) of m-phenoxybenzaldehyde was dissolved in 40 ml of methylene chloride, and chlorine gas was passed through the resulting solution at 0°C for 30 minutes. After the reaction was finished, the reaction solution was treated in the same manner as in Example 1 to give 11.54 g of m-(p-chlorophenoxy)benzaldehyde as a crude product. (No. i in Table 1).

### Example 1

9.90 Grams (0.050 mole) of m-phenoxybenzaldehyde was dissolved in 40 ml of methylene chloride, and a solution of 9.60 g (0.060 mole) of bromine in 10 ml of methylene chloride was added dropwise to the resulting solution at 0°C over 1 hour with stirring. After the addition was finished, the reaction was continued for further 3 hours at the same temperature. The reaction solution was washed with water and then with a dilute aqueous potassium sulfite solution to decompose bromine in the organic layer. The organic layer obtained was washed with a dilute aqueous sodium carbonate solution and then a dilute aqueous sodium chloride solution followed by drying over anhydrous sodium sulfate. The solvent was evaporated to give 12.95 g of m-(p-bromophenoxy)benzaldehyde as a crude product. b.p.: 130°—135°C (0.12 mmHg). (No. 2 in Table 2).

### Example 2

0.41 Gram (0.0025 mole) of anhydrous ferric chloride was added to a solution of 9.90 g (0.050 mole) of m-phenoxybenzaldehyde in 40 ml of methylene chloride to obtain a homogeneous solution. Thereafter, a solution of 9.60 g (0.060 mole) of bromine in 10 ml of methylene chloride was added dropwise to the solution at 0°C over 1 hour with stirring. The reaction solution was then treated in the same manner as in Example 1 to give 12.62 g of m-(p-bromophenoxy)benzaldehyde as a crude product (No. 3 in Table 2).

### Example 3

9.90 Grams (0.050 mole) of m-phenoxybenzaldehyde was dissolved in 40 ml of methylene chloride. Separately from this, 4.80 g (0.030 mole) of bromine and 1.78 g (0.025 mole) of chlorine were reacted at —20°C in methylene chloride to prepare a solution of bromine chloride in methylene

chloride. This solution was added dropwise to the above solution at –30°C over 30 minutes with stirring. After the addition was finished, the reaction was continued for further 30 minutes at the same temperature. The reaction solution was treated in the same manner as in Example 1 to give 13.29 g of m-(p-bromophenoxy)benzaldehyde as a crude product (No. 8 in Table 2).

Example 4

9.60 Grams (0.060 mole) of bromine was added dropwise to 9.90 g (0.050 mole) of m-phenoxybenzaldehyde at 0°C over 1 hour with vigorous stirring. After the addition was finished, the reaction was continued for further 3 hours at the same temperature. The unreacted bromine in the reaction mixture was evaporated under reduced pressure to give 13.14 g of m-(p-bromophenoxy)benzaldehyde as a crude product (No. 9 in Table 2).

TABLE 1: Chlorination *4 of m-phenoxybenzaldehyde (POAL)

| No. | Reaction temp. | Reaction time | Chlorinating agent *1 | Catalyst (Mole % based on POAL) | Solvent | Distribution (%) of products *2 | | | Pure yield *3 (%) |
|---|---|---|---|---|---|---|---|---|---|
| | | | | | | POAL | m-(o-chloro-phenoxy)-benzaldehyde | m-(p-chloro-phenoxy)-benzaldehyde | |
| i | 0°C | 0.5 hr | chlorine gas | – | $CH_2Cl_2$ | < 0.1 | 4.5 | 65.4 | 64.9 |
| ii | 20°C | 0.5 hr | chlorine gas | – | $CH_2Cl_2$ | < 0.1 | 3.5 | 57.4 | 59.1 |
| iii | -15°C | 0.5 hr | chlorine gas | $FeCl_3$ (5.0) | $CH_2Cl_2$ | < 0.1 | 8.5 | 75.7 | 73.8 |
| iv | -15°C | 0.5 hr | chlorine gas | $FeCl_3$ (2.5) | $CH_2Cl_2$ | < 0.1 | 7.7 | 76.2 | 72.2 |
| v | 0°C | 0.5 hr | chlorine gas | $FeCl_3$ (5.0) | $CH_2Cl_2$ | < 0.1 | 11.2 | 73.2 | 70.2 |
| vi | 0°C | 0.5 hr | chlorine gas | $ZnCl_2$ (5.0) | $CH_2Cl_2$ | < 0.1 | 8.9 | 73.0 | 70.1 |
| vii | 0°C | 0.5 hr | chlorine gas | $AlCl_3$ (5.0) | $CH_2Cl_2$ | < 0.1 | 8.1 | 67.3 | 62.3 |
| viii | -15°C | 0.5 hr | chlorine gas | $TiCl_4$ (5.0) | $CH_2Cl_2$ | < 0.1 | 5.8 | 70.6 | 68.3 |
| ix | -15°C | 0.5 hr | chlorine gas | $AlCl_3$ (100) | $CH_2Cl_2$ | < 0.1 | 17.6 | 58.2 | 55.5 |

Note: *1 Reaction was carried out by passing chlorine gas through a reaction mixture and was stopped at the disappearance of m-phenoxybenzaldehyde by gas-chromatographic analysis.

*2 Distribution was measured by gas-chromatography on the basis of the area of the products.

*3 Pure yield was determined by multiplying crude yield by the percentage of m-(p-chlorophenoxy)-benzaldehyde in the products.

*4 All of the experiments were carried out in the similar manner as in the Reference Example.

TABLE 2: Bromination *3 of m-phenoxybenzaldehyde (POAL)

| No. | Reaction temp. | Addition time | Reaction time | Brominating agent (Mole equivalent to POAL) | Catalyst (Mole % based on POAL) | Solvent | Distribution (%) of products *1 | | | Pure yield *2 (%) |
|---|---|---|---|---|---|---|---|---|---|---|
| | | | | | | | POAL | m-(o-bromo-phenoxy)-benzaldehyde | m-(p-bromo-phenoxy)-benzaldehyde | |
| 1 | 20°C | 1.0 hr | 3.0 hr | $Br_2$ (1.2) | – | $CH_2Cl_2$ | 0.1 | < 0.1 | 91.4 | 85.1 |
| 2 | 0°C | 1.0 hr | 3.0 hr | $Br_2$ (1.2) | – | $CH_2Cl_2$ | 0.1 | < 0.1 | 92.8 | 86.8 |
| 3 | 0°C | 1.0 hr | 3.0 hr | $Br_2$ (1.2) | $FeCl_3$ (5.0) | $CH_2Cl_2$ | 0.1 | < 0.1 | 92.3 | 84.1 |
| 4 | 0°C | 1.0 hr | 3.0 hr | $Br_2$ (1.2) | $AlCl_3$ (5.0) | $CH_2Cl_2$ | 0.1 | < 0.1 | 93.0 | 87.0 |
| 5 | 0°C | 1.0 hr | 3.0 hr | $Br_2$ (1.2) | $ZnCl_2$ (5.0) | $CH_2Cl_2$ | 0.1 | < 0.1 | 92.8 | 85.5 |
| 6 | 0°C | 1.0 hr | 3.0 hr | $Br_2$ (1.2) | – | $Cl_2C=CCl_2$ | 0.1 | < 0.1 | 92.5 | 86.3 |
| 7 | 0°C | 1.0 hr | 3.0 hr | $Br_2$ (1.2) | – | chlorobenzene | 0.1 | < 0.1 | 92.3 | 86.5 |
| 8 | -30°C | 0.5 hr | 0.5 hr | BrCl (1.0) | – | $CH_2Cl_2$ | 0.1 | < 0.1 | 84.9 | 81.5 |
| 9 | 0°C | 1.0 hr | 3.0 hr | Br (1.2) | – | – | 0.1 | < 0.1 | 88.1 | 83.6 |

Note: *1 Distribution was measured by gas-chromatography on the basis of the area of the products.

*2 Pure yield was determined by multiplying crude yield by the percentage of m-(p-bromophenoxy)-benzaldehyde in the products.

*3 All of the experiments were carried out in the similar manner as those described in Examples.

The manner in which the pure yield values given in Tables 1 and 2 are calcuated from the crude yield is illustrated by the examples given in the following Table 3.

TABLE 3

| Example | Crude yield (%) | | Area ratio (%) of m-(p-halophenoxy)benzaldehyde in gas chromatography | | Pure yield (%) |
|---|---|---|---|---|---|
| Reference Example 1 (Table 1, No. i) | 99.2 | × | 65.4/100 | = | 64.9 |
| Example 1 (Table 2, No. 2) | 93.5 | × | 92.8/100 | = | 86.8 |
| Example 2 (Table 2, No. 3) | 91.1 | × | 92.3/100 | = | 84.1 |
| Example 3 (Table 2 No. 8) | 96.0 | × | 84.9/100 | = | 81.5 |

## Claims

1. A process for preparing m-(p-bromophenoxy)benzaldehyde of the formula:

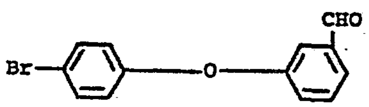

characterized in that m-phenoxybenzaldehyde is treated with bromine or bromine chloride.

2. A process according to claim 1, wherein the bromination is carried out in a halogenation-resistant solvent at a temperature lower than the boiling point of the solvent.

3. A process according to claim 2, wherein the bromination is carried out at a temperature of −30°C to 20°C.

4. A process according to any one of the preceding claims, wherein the molar ratio of the m-phenoxybenzaldehyde to the brominating agent is from 1:1 to 1:1.2.

## Patentansprüche

1. Verfahren zur Herstellung von m-(p-Bromphenoxy)benzaldehyd der Formel

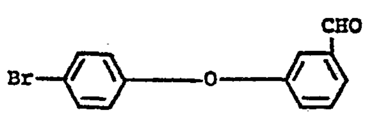

dadurch gekennzeichnet, daß man m-Phenoxybenzaldehyd mit Brom oder Bromchlorid behandelt.

2. Verfahren nach Anspruch 1, dadurch gekennzeichnet, daß die Bromierung in einem gegenüber der Halogenierung beständigen Lösungsmittel bei einer Temperatur durchgeführt wird, die unterhalb des Siedepunkts des Lösungsmittels liegt.

3. Verfahren nach Anspruch 2, dadurch gekennzeichnet, daß die Bromierung bei Temperaturen von −30°C bis 20°C durchgeführt wird.

4. Verfahren nach einem der vorstehenden Ansprüche, dadurch gekennzeichnet, daß das Molverhältnis von m-Phenoxybenzaldehyd zum Bromierungsmittel in einem Bereich von 1:1 bis 1:1,2 liegt.

## Revendications

1. Procédé de préparation du m-(p-bromophénoxy)-benzaldéhyde de formule

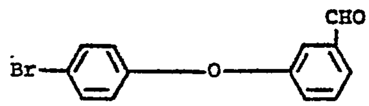

caractérisé en ce que l'on traite le m-phénoxybenzaldéhyde par le brome ou le chlorure de brome.

2. Procédé selon la revendication 1, caractérisé en ce que la bromation est effectuée dans un solvant résistant aux halogénations à une température inférieure au point d'ébullition du solvant.

3. Procédé selon la revendication 2, caractérisé en ce que la bromation est effectuée à une température comprise entre —30 et 20°C.

4. Procédé selon l'une quelconque des revendications qui précèdent, dans lequel le rapport molaire entre le m-phénoxybenzaldéhyde et l'agent bromant va de 1:1 à 1:1,2.